# EUROPEAN PATENT APPLICATION

(11) **EP 2 452 646 A1**
(43) Date of publication of application: **16.05.2012**
(21) Application number: 11188253.6
(22) Date of filing: 08.11.2011
(51) Int. Cl.: A61B 18/14, A61M 25/00, A61M 25/01

(54) **Device with electro-catheter for reducing a reversible neural lesion**

(30) Priority: 10.11.2010 IT MI20100341 U
(71) Applicant: Finella Medical S.p.A., 20124 Milan (IT)
(72) Inventor: Reverberi, Claudio, 20124 Milan (IT); Sluijter, Menno E., 20124 Milan (IT)
(74) Representative: Pietra, Giulia

(57) **Abstract**

It is disclosed a device for inducing a reversible neural lesion. The device comprises: an electro-catheter comprising an electrode at a distal end thereof; a control unit; and two control wires housed in the electro-catheter and having a first end fixed in the electrode and a second end fixed in the control unit. The control unit, when operated, applies a traction to one of the two control wires, which transmits the traction to the electrode, thereby inducing a rotation of the electrode in a plane which contains the two control wires. The two control wires are also suitable for supplying the electrode with an electric current.

## Description

The present invention generally relates to the field of electro-medical devices. In particular, the present invention relates to a device with an electro-catheter suitable for inducing a reversible neural lesion to reduce or eliminate a benign pain.

In the art, electro-medical devices are known that allow performing treatments aimed at reducing or eliminating pain due to benign pathologies, such as for instance lumbago, lumbosciatica, disc hernia, etc.

These electro-medical devices typically comprise an electro-catheter, i.e. a catheter provided with an electrode in the vicinity or at his distal end.

The distal end of the electro-catheter is for instance inserted into the spine's peridural space of the patient to undergo treatment. Once the peridural space is reached, the distal end of the electro-catheter is positioned in the proximity of the spinal root and the respective posterior dorsal ganglion, at the point in which they exit the intervertebral foramen into the lateral epidural recesses.

During the insertion of the electro-catheter, the patient is typically subject to an X-ray examination, in order to allow the medical staff to precisely check the movements and the position of the distal end of the electro-catheter.

Once in position, the electro-catheter is connected to an external radio frequency electric generator suitable for providing an alternating electric current to the electrode of the electro-catheter. The electric current applied to the spinal cord via the spinal root and the posterior dorsal ganglion induces a reversible neural lesion, which temporarily interrupts the conduction of the electric signals which transmit the sensation of pain to the brain.

The Italian patent application for a utility model No. M12009U000106, filed on April 2, 2009 in the name of the same Applicant, discloses a device for inducing a reversible epidural lesion which comprises an electro-catheter, a control unit and two control wires having a first end fixed in the proximity of the distal end of the electro-catheter and a second end fixed in the control unit. The control unit, when operated, applies a traction to one of the two control wires, which transmits the traction to the distal end, inducing a bending of the electro-catheter. The electro-catheter of MI2009U000106 further comprises a conductive wire electrically connected to the electrode and also a thermocouple. The external diameter of the electro-catheter is of about 1.7 mm.

The inventors noticed that the electro-catheter disclosed by M12009U000106 has been expressly designed for use in the peridural space and that it may be not suitable for use in narrower spaces than the peridural space, such as for instance the intra-articular spaces and the inter-disc spaces. Indeed, in such narrower spaces it is preferable to use electro-catheters with a much reduced diameter, in order to minimize the risk of inducing unwanted lesions of the central or peripheral nervous system of the patient (with the risk that the patient suffers temporary or even permanent neurological deficiencies) or of causing the formation of haematomas (which should be removed by surgical drainage).

Therefore, the inventors faced the problem of providing a device with electro-catheter suitable for inducing a reversible neural lesion to reduce or eliminate a benign pain, wherein the electro-catheter has a reduced external diameter, so that during its usage (in the peridural space, but also in narrower spaces such as for instance intra-articular spaces and inter-disc spaces) the risk of unwanted lesions to the patient's nervous system and/or the formation of haematomas is minimized.

According to embodiments of the present invention, it is provided a device for inducing a reversible neural lesion, the device comprising:
- an electro-catheter comprising an electrode at a distal end thereof;
- a control unit; and
- two control wires housed in the electro-catheter and having a first end fixed in the electrode and a second end fixed in the control unit,
wherein the control unit, when operated, applies a traction to one of the two control wires, which transmits the traction to said electrode, thereby inducing a rotation of the electrode in a plane which contains the two control wires, and wherein the two control wires are also suitable for supplying the electrode with an electric current.

Preferably, the electro-catheter comprises an elongated tubular element and a joint that connects the electrode to a distal end of the elongated tubular element.

Preferably, the electro-catheter further comprises:
- two first guiding tubes housed in the elongated tubular element, a first portion of the two control wires being housed in the two first guiding tubes; and
- two second guiding tubes housed in the electrode, a second portion of the two control wires being housed in the two second guiding tubes.

According to preferred embodiments, the joint has a lateral wall that defines a central cavity and at least one wall that divides that central cavity in at least two sectors, a third portion of the two control wires intermediate between the first portion and the second portion being housed in the at least two sectors.

Preferably, the two first guiding tubes are in symmetric positions relative to a longitudinal axis of the electro-catheter, and also the two second guiding tubes are in symmetric positions relative to the longitudinal axis of the electro-catheter.

Preferably, the two first guiding tubes protrude from a distal end of the elongated tubular element by a first length and engage a proximal end of the joint, and the two second guiding tubes protrude from a proximal end of the electrode by a second length and engage a distal end of the joint.

Preferably, the sum of the first length and the second length is shorter than the length of the joint.

Preferably, a length of the control wires comprised between the two first guiding tubes and the two second guiding tubes is coated by an insulating material.

Preferably, the first guiding tubes and the second guiding tubes are made of metal.

Preferably, the elongated tubular element is made of a first plastic material.

According to preferred embodiments, the joint is made of a second plastic material having a flexibility greater than the first plastic material.

Preferably, the electrode is made of stainless steel.

Preferably, the two control wires are made of stainless steel.

According to advantageous embodiments, the electro-catheter further comprises an injection duct having a distal end connected with the exterior of the electrode and a proximal end fixed in the control unit.

Preferably, the control unit comprises a screw and two pins engaging the screw, the second end of each of the two control wires being wound on a respective pin so that, when the screw rotates, one of the two control wires is further wound on the respective pin.

The invention will be clearer from the following description, given by way of example and not of limitation, to be read with reference to the accompanying drawings, wherein:
- Figure 1 is a plan view from above of a device according to an embodiment of the present invention;
- Figures 2a-2e are five cross sections of the distal end of the electro-catheter at five cross sectional planes A-E whose projections are shown in Figures 3 and 4;
- Figure 3 is a longitudinal section of the distal end of the electro-catheter along a first longitudinal plane y, whose projection is shown in Figure 2a-2e;
- Figure 4 is a longitudinal section of the distal end of the electro-catheter along a second longitudinal plane z perpendicular to the first longitudinal plane y, whose projection is also shown in Figures 2a-2e;
- Figure 5 is a perspective view of the control unit of the device of Figure 1;
- Figure 6 is a perspective view of a portion of the device of Figure 1 during its assembly; and
- Figure 7 is a plan view from above of a portion of the device of Figure 1 during its assembly.

With reference to the accompanying Figures, it will be now described a device according to an embodiment of the present invention. In the

Figures, similar parts will be designated with the same reference number.

The device 1 comprises an electro-catheter 2, a control unit 3 and a supply unit 4.

The electro-catheter 2 preferably comprises an elongated tubular element 21, a joint 22 and an electrode 23.

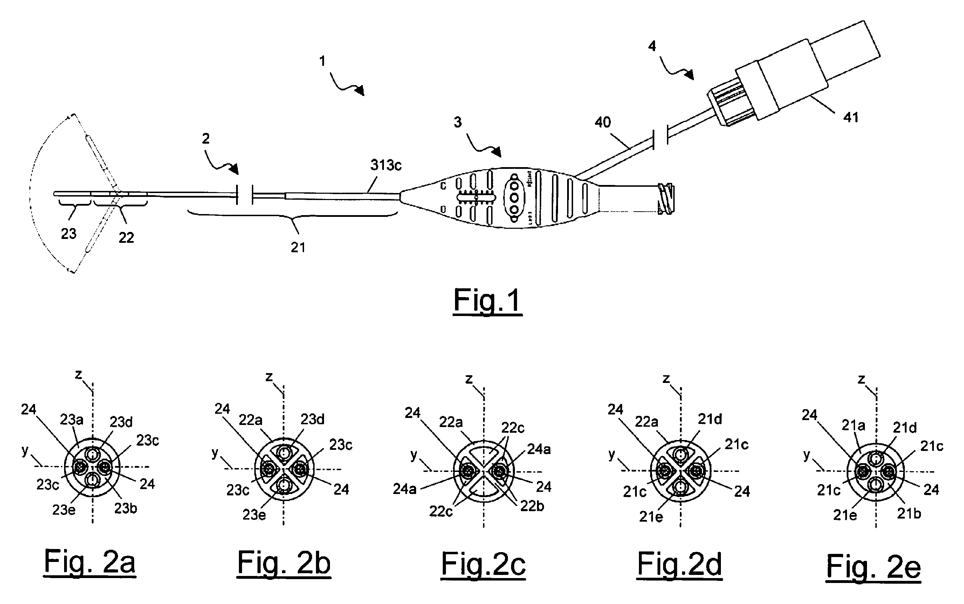

With reference to Figures 2e, 3 and 4, the elongated tubular element 21 preferably has a lateral wall 21 a that defines a central cavity 21 b of substantially circular cross-section, which extends along the whole elongated tubular element 21.

The elongated tubular element 21 preferably has an external diameter comprised between 0.7 mm and 1.5 mm, more preferably equal to 1.1 mm. The central cavity 21 b preferably has a diameter comprised between 0.5 mm and 1.3 m, more preferably equal to 0.9 mm.

Preferably, the elongated tubular element 21 is made of a flexible plastic material, such as for instance a polyamide (e.g. Grilamid™ L25).

Preferably, a graduated metric scale is printed on the external surface of the lateral wall 21 a of the elongated tubular element 21. Preferably, the graduated metric scale is printed (for instance, by an inkjet printer) during the operation of extrusion of the elongated tubular element 21. The graduated metric scale advantageously allows monitoring in a simple and fast way the level of insertion of the electro-catheter 2.

The elongated tubular element 21 has a proximal end inserted into the control unit 2 and a distal end connected to the electrode 23 through the joint 22, as it will be described in greater detail herein after.

The central cavity 21 b of the elongated tubular element 21 preferably houses two first guiding tubes 21 c, a first injection tube 21 d and a first additional tube 21e having a preferably circular cross-section and arranged parallel with the longitudinal axis of the elongated tubular element 21. Preferably, as shown in Figure 2e, the two first guiding tubes 21 c are in symmetric positions relative to the longitudinal axis of the elongated tubular element 21 and they have their respective centres on a first longitudinal plane y of the tubular body 21. Further, as shown in Figure 2e, the first injection tube 21 d and the first additional tube 21 e are preferably in symmetric positions relative to the longitudinal axis of the elongated tubular element 21 and they have their respective centres on a second longitudinal plane z of the tubular body 21, perpendicular to the first plane y.

The two first guiding tubes 21 c, the first injection tube 21 d and the first additional tube 21 e preferably have an external diameter comprised between 0.30 mm and 0.40 mm, more preferably equal to about 0.36 mm. Besides, the two first guiding tubes 21 c, the first injection tube 21 d and the first additional tube 21e preferably have an internal diameter comprised between 0.15 mm and 0.25 mm, more preferably equal to about 0.20 mm.

The two first guiding tubes 21 c, the first injection tube 21 d and the first additional tube 21 e preferably extend along the whole length of the elongated tubular element 21 and they protrude from its distal end. In particular, they protrude from the distal end of the elongated tubular element 21 by a length preferably comprised between 2 mm and 6 mm, more preferably equal to 4 mm.

The two first guiding tubes 21 c, the first injection tube 21 d and the first additional tube 21e are preferably made of metal, more preferably of stainless steel (e.g. AISI 304).

With reference now to Figures 2b-2d, 3 and 4, the joint 22 preferably has a lateral wall 22a substantially cylindrical that defines a central cavity and two longitudinal walls 22b that intersect substantially at right angles (as shown in Figures 2b, 2c and 2d) so as to divide the central cavity in four sectors 22c having substantially the same section.

Preferably, the joint 22 has an external diameter comprised between 0.8 mm and 1.6 mm, more preferably equal to 1.17 mm. Preferably, the joint 22 has a length comprised between 9 mm and 17 mm, more preferably equal to 13 mm.

The joint 22 is preferably made of a flexible plastic material having a greater flexibility than the plastic material of the elongated tubular element 21. The joint 22 may be made of a polyamide such as for instance Grilflex ™ ELG 3660. Using two plastic materials with different flexibility advantageously allows providing the electro-catheter 2 with an adequate stiffness so that it may be inserted into the peridural space without bending, and moreover guarantees the possibility of rotating the electrode 23 in the first longitudinal plane y by operating the control unit 3 (as it will be explained in greater detail herein after) so that the joint 22 only is bent, without inducing any bending of the elongated tubular element 21.

With reference now to Figures 2a, 3 and 4, the electrode 23 preferably has a lateral wall 23a substantially cylindrical that defines a central cavity 23b, and it has an open proximal end and a substantially closed and rounded distal end.

The electrode 23 preferably has an external diameter comprised between 0.7 mm and 1.5 mm, more preferably equal to 1.1 mm. The central cavity 23b preferably has a diameter comprised between 0.4 mm and 1.2 mm, more preferably equal to 0.8 mm. The electrode 23 preferably has a length comprised between 6 mm and 14 mm, more preferably equal to 10 mm.

The electrode 23 is preferably made of metal, more preferably of stainless steel (e.g. AISI 304).

Preferably, the electrode 23 ha an echo-generating coating. For instance, to obtain the echo-generating coating, the electrode 23 ma be subjected to caulking. The echo-generating coating advantageously allows performing the operation of inserting and positioning the electro-catheter 2 while the patient is subject to ultrasounds which, contrary to X-ray examination, are harmless for the patient and for the medical staff who is performing the operation.

The central cavity 23b of the electrode 23 preferably houses two second guiding tubes 23c, a second injection tube 23d and a second additional tube 23e having a preferably circular cross-section and arranged in parallel with the longitudinal axis of the electrode 23. Preferably, as shown in Figure 2a, the two second guiding tubes 23c are in symmetric positions relative to the longitudinal axis of the electrode 23 and they have their respective centres on the first longitudinal plane y. Further, as shown in Figure 2a, the second injection tube 23d and the second additional tube 23e are preferably in symmetric positions relative to the longitudinal axis of the electrode 23 and they have their respective centres on the second longitudinal plane z.

The two second guiding tubes 23c, the second injection tube 23d and the second additional tube 23e preferably have an external diameter comprised between 0.30 mm and 0.40 mm, more preferably equal to about 0.36 mm. Besides, the two second guiding tubes 23c, the second injection tube 23d and the second additional tube 23e preferably have an internal diameter comprised between 0.15 mm and 0.25 mm, more preferably equal to about 0.20 mm.

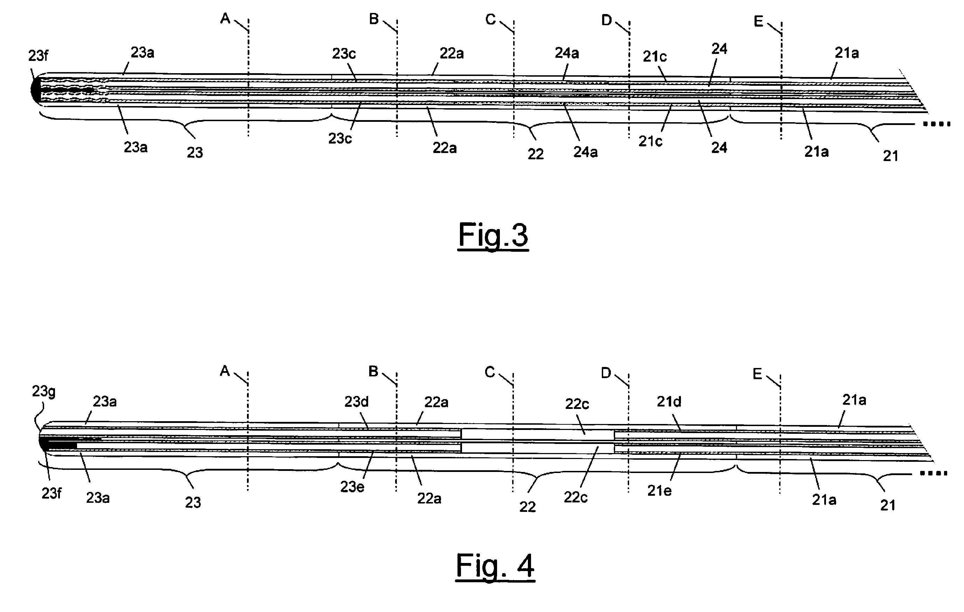

The two second guiding tubes 23c, the second injection tube 23d and the second additional tube 23e preferably have an end fixed to the internal surface of the substantially closed and rounded distal end of the electrode 23, for instance by means of a welding 23f shown in Figures 3 and 4. At the second injection tube 23d, the distal end of the electrode 23 preferably has a hole 23g, through which the second injection tube 23d is communicating with the exterior of the electrode 23.

The two second guiding tubes 23c, the second injection tube 23d and the second additional tube 23e extend along the whole length of the electrode 23 and they protrude from its open proximal end. In particular, they protrude from the open proximal end of the electrode 23 by a length preferably comprised between 2 mm and 6 mm, more preferably equal to 4 mm.

Also the two second guiding tubes 23c, the second injection tube 23d and the second additional tube 23e are preferably made of metal, more preferably of stainless steel (e.g. AISI 304).

As mentioned above, the distal end of the elongated tubular element 21 is connected to the electrode 23 through the joint 22.

In particular, with reference to Figures 2b, 2d, 3 and 4, the proximal ends of two opposite sectors 22c of the joint 22 are preferably engaged by the lengths of the two first guiding tubes 21 c that protrude from the elongated tubular body 21 (see Figure 2d) and the distal ends are engaged by the lengths of the two second guiding tubes 23c that protrude from the electrode 23 (see Figure 2b). This way, advantageously, two substantially parallel guiding paths are formed along the whole electro-catheter 2.

Further, the proximal end of a third sector 22c of the joint 22 is preferably engaged by the length of the first injection tube 21 d that protrudes from the elongated tubular body 21 (see Figure 2d) and the distal end is engaged by the length of the second injection tube 23d that protrudes from the electrode 23 (see Figure 2b). This way, advantageously, an injection path is formed along the whole electro-catheter 2.

Moreover, the proximal end of a fourth sector 22c of the joint 22 is preferably engaged by the length of the first additional tube 21e that protrudes from the elongated tubular body 21 (see Figure 2d) and the distal end is engaged by the length of the second additional tube 23e that protrudes from the electrode 23 (see Figure 2b). This way an additional path is formed along the whole electro-catheter 2.

The lateral wall 22a of the joint 22 is thus in contact at its proximal end with the lateral wall 21 a of the elongated tubular body 21, and at its distal end with the lateral wall 23a of the electrode 23.

Preferably, the sum of the length by which the first tubes 21 c, 21 d and 21 e protrude from the elongated tubular element 21 and the length by which the second tubes 23c, 23d and 23e protrude from the electrode 23 is smaller than the length of the joint 22. For instance, the joint may be 13 mm long, the first tubes 21 c, 21 d and 21e may protrude from the elongated tubular element 21 by 4 mm and the second tubes 23c, 23d and 23e may protrude from the electrode 23 by 4 mm. This way, advantageously, a central portion (5 mm long, according to the present example) of the joint 22 has no tubes, and it is thus much more flexible than the elongated tubular element 21 (that, apart from being of a more rigid plastic material, has also on the inside the first tubes 21 c, 21 d and 21e that increase its stiffness). This advantageously allows rotating the electrode 23 in the first longitudinal plane y bending the joint 22 only (in particular, its central portion having no tubes), as it will be described in greater detail herein after.

Preferably, the electro-catheter 2 further comprises two control and supply wires 24. Preferably the two control and supply wires 24 extend along the whole length of the electro-catheter 2, from the distal end of the electrode 23 to the proximal end of the elongated tubular element 21, from which they come out to be fixed in the control unit 3, as it will be described in greater detail herein after.

More in particular, each of the two control wires 24 is housed in one of the two substantially parallel guiding paths formed by the two first guiding tubes 21 c, by the two second guiding tubes 23c and by the joint 22. In the central portion of the joint 22 (which has no tubes), the control and supply wires 24 engage two opposite sectors 22s substantially without the protection of any tube, as shown in Figure 3.

The length of the control and supply wires 24 that engage the portion of the joint 22 having no tubes preferably has as insulating coating 24a. The insulating coating 24a is preferably of a flexible plastic material, such as a polyamide (e.g. Grilamid™ L25). The coating 24a preferably has a thickness comprised between 0.05 mm and 0.15 mm, more preferably equal to 0.1 mm. The two ends of the insulating coatings 24a are connected, for instance by thermal welding, to the ends of the guiding tubes 21 a and 23a.

At the distal end of the electrode 23, the control and supply wires 24 are preferably fixed to the respective second guiding tubes 23c, for instance by crimping or by welding.

The control and supply wires 24 have a substantially circular section, with a diameter preferably between 0.1 mm and 0.2 mm, more preferably equal to 0.15 mm. Preferably, the control and supply wires 24 are of stainless steel (e.g. AISI 316).

The supply unit 4 preferably comprises a radio-frequency conductor 40 and a radio-frequency connector 41. The radio-frequency conductor 40 is preferably coated by a sheath of an insulating material, e.g. a polyamide.

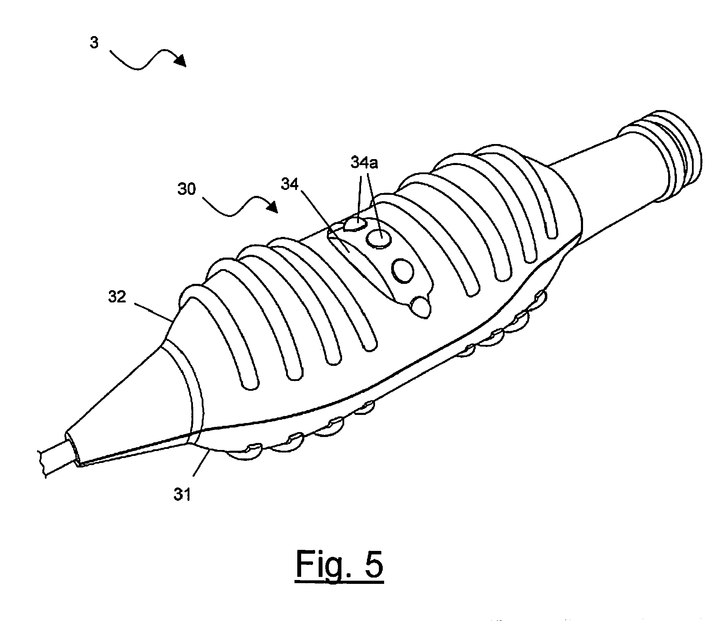

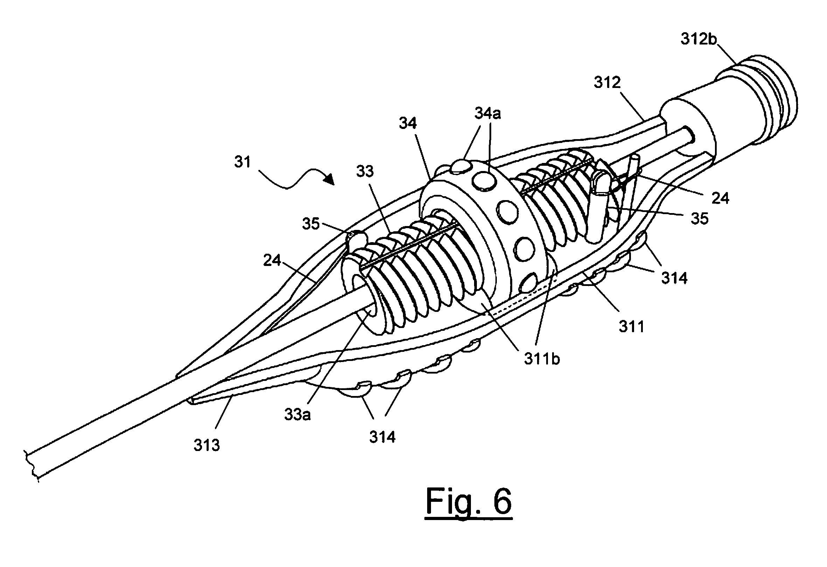

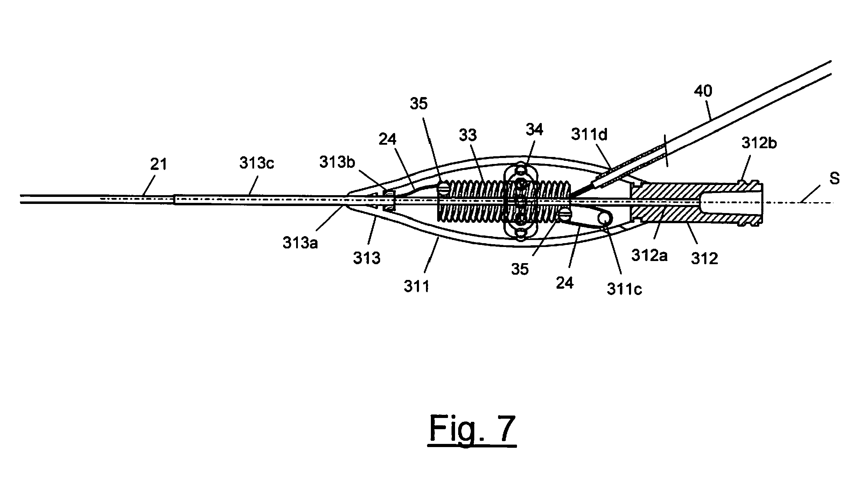

With reference now to Figures 5-7, the control unit 3 preferably comprises a casing 30, an endless screw 33, a control ring 34 and two winding pins 35.

The casing 30 is substantially in the form of a spindle with rotational symmetry about an axis S. The casing 30 is made up by a lower half-shell 31 and an upper half-shell 32.

The lower semi-shell 31 has an enlarged central portion 311 and two tapered proximal and distal end portions 312, 313.

The proximal end portion 312 is shaped so as to form a conic injection connection. The conic injection connection is preferably of the type Luer Lock, and it has a central duct 312a and an external thread 312b.

The distal end portion 313 preferably forms a tapered portion 313a suitable for receiving the proximal end of the electro-catheter 2, and has a guide 313b suitable for axially blocking the electro-catheter 2.

The central portion 311 of the lower half-shell 31 preferably has a slit (not shown in the drawings) arranged in a transverse direction with respect to the axis S. The edges of the slit preferably form two protrusions 311 b protruding towards the interior of the lower half-shell 31. Optionally, the central portion 311 may further comprise a fixed pin 311c placed in the proximity of the proximal end portion 312. The central portion 311 moreover preferably has a lateral recess 311 d suitable for receiving the conductor 40 of the supply unit 4, as it will be described in greater detail herein after.

The external surface of the lower half-shell 31 preferably has gripping relieves 134 which advantageously improve the grip on the casing 30 by an operator that holds the control unit 3 to use the device 1 on a patient.

The upper half-shell 32 is substantially similar to the lower half-shell 31, therefore it will not be described in detail. However, differently from the lower half-shell 31, the upper half-shell 32 has not the proximal end portion that forms the conic injection connection.

Preferably, the casing 30 has a length comprised between about 5 cm and about 9 cm, more preferably equal to about 7 cm. Further, preferably, the casing has a maximum diameter comprised between about 2 cm and 6 cm, more preferably equal to about 4 cm.

The lower half-shell 31 and the upper half-shell 32 are preferably moulded from a rigid plastic material, such as for instance ABS (Acrylonitrile Butadiene Styrene) with certificate of biocompatibility (e.g. Telux 2812).

The endless screw 33 preferably has an axial through hole 33a that crosses it along its whole length. Also the endless screw 33 is preferably moulded from a rigid plastic material. More preferably, the endless screw 33 is moulded from the same plastic material as the casing 30.

The control ring 34 is preferably a toroidal ring having an internal diameter slightly larger than the external diameter of the endless screw 33, and having on its internal surface a thread (non visible in the drawings). Further, on its external surface, the control ring 34 has non-skid relieves 34b suitable for facilitating the operation of rotating the control ring 34 when it is used to control the orientation of the electrode 23, as it will be explained in greater detail herein after. Also the control ring 34 is preferably of the same plastic material of the casing 30.

The winding pins 35 are preferably of polyamide.

During the assembly of the device of Figure 1, the winding pins 35 are preferably fixed to the lower half-shell 31 so that they protrude towards the interior of the lower half-shell 31, and that each of them is free of rotating about its axis. Preferably, the two winding pins 35 are placed on opposite sides of the axis S, the one in the proximity of the proximal end portion 312 and the other in the proximity of the distal end portion 313.

Then, the endless screw 33 is preferably inserted into the control ring 34, so that the thread of the endless screw 33 engages the internal thread (not visible in the drawings) of the control ring 34. The endless screw 33 with the control ring 34 is then preferably leaned on the lower half-shell 31 between the two winding pins 35, so that also each winding pin 35 engages the thread of the endless screw 33 and the control ring 34 engages the space between the protrusions 311b, partially protruding to the exterior of the lower half-shell 31 through the transverse slit (non shown in the drawings).

The proximal end of the electro-catheter 2 (i.e. of the elongated tubular element 21) is then leaned on the lower half-shell 31, so that it comes out through the tapered portion 313a (possibly through an adapter 313c). Preferably, the portion of the electro-catheter 2 which comes out from the control unit 3 has a length comprised between about 20 cm and about 100 cm. According to a first preferred embodiment, the portion of the electro-catheter 2 which comes out from the control unit 3 has a length of about 30 cm. According to a second preferred embodiment, the portion of the electro-catheter 2 which comes out from the control unit 3 has a length of about 60 cm.

Also the supply unit 4 is preferably leaned on the lower half-shell 31, so that the conductor 40 comes out from the lateral recess 311d. Preferably, the portion of the conductor 40 which comes out from the control unit 3 has a length of some tenths of centimetres, for example 15 cm.

The first guiding tubes 21 c, the first injection tube 21 d and the first additional tube 21e preferably come out from the proximal end of the elongated tubular element 21 leaned on the lower half-shell 31. One of the two first guiding tubes 21 c, the first injection tube 21 d and the first additional tube 21e are preferably inserted into the axial through hole 33a of the endless screw 33.

The ends of the control and supply wires 24 are extracted from the respective first guiding tubes 21 c, each of them is preferably wound on a respective winding pin 35 and finally they are electrically connected to the end of the conductor 40.

In particular, the free ends of the control and supply wires 24 are wound on the pins 35 according to opposite winding directions (i.e. one clockwise and one counter-clockwise) so that, when the endless screw 33 rotates inducing a rotation in the same rotation direction of both the winding pins 35 (e.g. clockwise), one of the two wires 24 is further wound on the respective pin 35, while the other wire 24 is partially unwound.

Preferably, each of the wires 24, once they have been wound, is fixed to the respective pin 35. To this aim, the winding pins 35 may comprise a head with a groove into which the wire 24 may be inserted and then glued (for example, with loctite® 4011 of medical type).

The proximal end of the first injection tube 21 d is then connected to the central duct 312a of the injection connection.

The upper half-shell 32 is then closed onto the lower half-shell 31, and the edges of the two half-shells are welded or glued one to the other. In case of gluing, it is preferably used the loctite® 4011. Advantageously, the transverse slits of the lower half-shell 31 and the upper half-shell 32 leave two opposite portions of the external surface of the control ring 34 with the relative non-skid relieves 34a exposed.

As mentioned above, the device 1 is suitable for inducing a reversible neural lesion to reduce or eliminate a benign pain. In particular, in order to induce a reversible epidural neural lesion in a patient, the distal end of the electro-catheter 2 is inserted in an peridural, intra-articular or inter-disc space of the patient, for example using an insertion device such as a needle.

Once reached the space, it is possible to precisely position the electrode 23 by rotating it in the longitudinal plane y on which the control and supply wires 24 are positioned.

This operation may be performed by holding the control unit 3 with the fingers of a hand and acting with one of the fingers on the relieves 34a of the control ring 34 that protrude through the transverse slits of the casing 30, so as to rotate the control ring 34.

The rotation of the control ring 34 induces a rotation of the endless screw 33, that in its turn transmits the rotation to the two winding pins 35. In particular, as mentioned above, the two pins 35 with the wires 24 wound thereon rotate in a same rotation direction (e.g. clockwise), so that one of the two wires 24 is further wound, while the other is partially unwound. Since the distal ends of the two wires 24 are fixed to the electrode 23 at its distal end, the wire 24 that is further wound is subject to a traction that advantageously induces a rotation of the electrode 23 towards the wire 24 which is pulled.

The electrode 23 may advantageously be rotated by a maximum angle equal to ±45° relative to the longitudinal axis of the elongated tubular element 21 (see Figure 1).

Since the electro-catheter 2 has, at the joint 22 (and, in particular, at the central portion of the joint 22 that does not contain any metal tube), a flexibility much greater than at the electrode 23 (which is of metal) and the elongated tubular element 21 (which is of a more rigid plastic material, and moreover contains metal tubes), the bending of the distal end of the electro-catheter 2 practically is concentrated in the central portion of the joint 22, as shown in Figure 1. In other words, the joint 22 bends and allows the rotation of the electrode 23, while the elongated tubular element 21 remains advantageously substantially straight.

The treatment is completed by connecting the supply unit 4 to a radio-frequency generator through the radio-frequency connector 41. This way, the conductor 40 transfers the electric current produced by the generator to the control and supply wires 24, which in their turn transmit the current to the electrode 23, which produces the reversible neural lesion.

At the same time, it is possible to use the injection connection of the control unit 3 to inject (through the duct 312a and the injection path inside the electro-catheter 2) a liquid in the area surrounding the electrode 23 (for example, anaesthetic, contrast liquid, analgesic, etc.).

The device described above has thus several advantages.

Firstly, thanks to the fact that the control and supply wires 24 are used both for controlling the bending of the distal end of the electro-catheter 2 and for electrically supplying the electrode 23, it is not necessary to provide the electro-catheter with a separate conductor. Therefore, the diameter of the electro-catheter 2 may advantageously be very reduced, so that the device 1 is suitable for being used not only in peridural spaces, but also in narrower spaces, such as intra-articular spaces and inter-disc spaces.

The bending of the distal end of the electro-catheter 2 may advantageously be controlled in a very precise manner, even if the diameter of the electro-catheter 2 is very reduced (e.g. 1.1 mm). This depends from the fact that the control and supply wires 24 are guided by the parallel guiding paths formed by the guiding tubes 21 c, 23c and by the sectors 22c of the joint 22 along the whole length of the joint 22, until the tip of the electrode 23. This allows maintaining the wires 24 at a substantially constant distance along the whole electro-catheter 2, and in particular at the tip of the electrode 23.

To this aim, the inventors observed that the presence of the additional tubes 21 e, 23e advantageously improves the control accuracy of the bending of the distal end of the electro-catheter 2. The inventors maintain that this is due to the fact that the additional tubes 21 e, 23e provide symmetry to the section of the electro-catheter 2.

Advantageously, moreover, the control unit 3 is very compact and light. This allows the electro-catheter 2 to support the weight of the control unit 3, notwithstanding the very reduced diameter.

## Claims

1. A device (1) for inducing a reversible neural lesion, said device (1) comprising:
- an electro-catheter (2) comprising an electrode (23) at a distal end thereof;
- a control unit (3); and
- two control wires (24) housed in said electro-catheter (2) and having a first end fixed in said electrode (23) and a second end fixed in said control unit (3),
wherein said control unit (3), when operated, applies a traction to one of said two control wires (24), which transmits said traction to said electrode (23), thereby inducing a rotation of said electrode (23) in a plane which contains said two control wires (24), and wherein said two control wires (24) are also suitable for supplying said electrode (23) with an electric current.

2. The device (1) according to claim 1, wherein said electro-catheter (2) comprises an elongated tubular element (21) and a joint (22) that connects said electrode (23) to a distal end of said elongated tubular element (21).

3. The device (1) according to claim 2, wherein said electro-catheter (2) further comprises:
- two first guiding tubes (21 c) housed in said elongated tubular element (21), a first portion of said two control wires (24) being housed in said two first guiding tubes (21 c); and
- two second guiding tubes (23c) housed in said electrode (23), a second portion of said two control wires (24) being housed in said two second guiding tubes (23c).

4. The device (1) according to claim 3, wherein said joint (22) has a lateral wall (22a) that defines a central cavity and at least one wall (22b) that divides that central cavity in at least two sectors (22c), a third portion of said two control wires (24) intermediate between said first portion and said second portion being housed in said at least two sectors (22c).

5. The device (1) according to claim 3 or 4, wherein:
- said two first guiding tubes (21 c) are in symmetric positions relative to a longitudinal axis of said electro-catheter (2); and
- said two second guiding tubes (23c) are in symmetric positions relative to said longitudinal axis of said electro-catheter (2).

6. The device (1) according to any of claims 3 to 5, wherein:
- said two first guiding tubes (21 c) protrude from a distal end of said elongated tubular element (21) by a first length and engage a proximal end of said joint (22); and
- said two second guiding tubes (23c) protrude from a proximal end of said electrode (23) by a second length and engage a distal end of said joint (22).

7. The device (1) according to claim 6, wherein a sum of said first length and said second length is shorter than a length of said joint (22).

8. The device (1) according to claim 7, wherein a length of said control wires (24) comprised between said two first guiding tubes (21 c) and said two second guiding tubes (23c) is coated by an insulating material.

9. The device (1) according to any of claims 3 to 8, wherein said first guiding tubes (21 c) and said second guiding tubes (23c) are made of metal.

10. The device (1) according to any of claims 2 to 9, wherein said elongated tubular element (21) is made of a first plastic material.

11. The device (1) according to claim 10, wherein said joint (22) is made of a second plastic material having a flexibility greater than said first plastic material.

12. The device (1) according to any of the preceding claims, wherein said electrode (23) is made of stainless steel.

13. The device (1) according to any of the preceding claims, wherein said two control wires (24) are made of stainless steel.

14. The device (1) according to any of the preceding claims, wherein said electro-catheter (2) further comprises an injection duct (21 d, 23d) having a distal end connected with the exterior of said electrode (23) and a proximal end fixed in said control unit (3).

15. The device (1) according to any of the preceding claims, wherein said control unit (3) comprises a screw (33) and two pins (35) engaging said screw (33), said second end of each of said two control wires (24) being wound on a respective pin (35) so that, when said screw (33) rotates, one of said two control wires (24) is further wound on the respective pin (35).
